# EUROPEAN PATENT SPECIFICATION

(11) **EP 3 305 185 B1**
(45) Date of publication and mention of the grant of the patent: **30.10.2024**
(21) Application number: 15845493.4
(22) Date of filing: 11.11.2015
(51) Int. Cl.: A61B 5/021

(54) **SPHYGMOMANOMETER**
SPHYGMOMANOMETER
SPHYGMOMANOMÈTRE

(30) Priority: 26.05.2015 CN 201510275205
(43) Date of publication of application: 11.04.2018
(73) Proprietor: BOE Technology Group Co., Ltd., Beijing 100015 (CN)
(72) Inventor: YUAN, Zuo, Beijing 100176 (CN)
(74) Representative: Klunker IP Patentanwälte PartG mbB
(86) International application number: PCT/CN2015/094323
(87) International publication number: WO 2016/188048

(56) References cited:
- WO-A1-2014/089665
- WO-A2-2008/057884
- CN-A- 1 762 300
- CN-A- 1 923 136
- CN-A- 103 371 811
- CN-A- 104 840 189
- CN-U- 204 671 137
- US-A1- 2013 281 868
- US-A1- 2013 296 723
- US-A1- 2014 031 662

## Description

### FIELD

The present invention refers to a device for measuring blood pressure.

### BACKGROUND

Blood pressure is a very important physiological parameter of human body. Regular measure of blood pressure is advantageous for earlier detection and diagnosis of the variety of hypertension disease so as to propose appropriate therapeutic recommendation thereto.

At present, existing devices for measuring blood pressure usually comprise standard mercury sphygmomanometer and electronic sphygmomanometer. Practical operation of the standard mercury sphygmomanometer requires a user to possess certain specialized knowledge, which results in relatively narrower application range. The electronic sphygmomanometer doesn't require the user to possess much specialized knowledge for practical operation, and hence is relatively broader in application range. However, both of the standard mercury sphygmomanometer and the electronic sphygmomanometer require the user to wear a wrist strap belt, with relatively complicated operation process; moreover, the standard mercury sphygmomanometer and the electronic sphygmomanometer both have relatively larger size, which makes the miniaturization and portability of the sphygmomanometer unachievable and cannot satisfy demand of user who requires for measurement of blood pressure whenever and wherever possible.

Therefore, the present filed always has a demand for devices for measuring blood pressure with simple operation and small size for portability.

CN 1 762 300 A discloses a device for measuring heart rate and blood pressure by employing the photo volume marking signal of the body and combining the high-frequency photo volume marking signal with the electrocardiosignal to measure the body physiological parameter.

US 2013/296723 A1 discloses an apparatus for readily measuring a blood pressure without using a cuff including measuring, by a portable blood pressure measuring apparatus, an electrocardiogram signal and a pulse wave signal, transmitting the measured electrocardiogram signal and pulse wave signal to a portable terminal, calculating, by the portable terminal, a Pulse Transit Time (PTT) and a Pulse Wave Velocity (PWV) using the transmitted electrocardiogram signal and the pulse wave signal, and calculating a blood pressure value based on the PTT and the PWV.

US 2013/281868 A1 discloses a blood pressure measurement device including a case, an electrocardiogram electrode, a pulse wave sensor, an estimation portion, and a display portion. The case has a peripheral surface to be held with both hands. The electrocardiogram electrode detects an electrocardiogram signal associated with a movement of a heart through at least one of the hands. The pulse wave sensor detects a pulse wave signal associated with the movement of the heart through a least one of the hands. The estimation portion estimates a blood pressure based on the electrocardiogram signal and the pulse wave signal.

WO 2014/089665 A1 discloses a system that continuously monitors cardiovascular health using an electrocardiography (ECG) source synchronized to an optical (PPG) source, without requiring invasive techniques or ongoing, large-scale external scanning procedures.

WO 2008/057884 A2 discloses a body worn physiological sensor device having a disposable electrode module.

### SUMMARY

It is an object of the present intention to provide a device for measuring blood pressure achieving simple operation and small size for portability.

The object is achieved by the features of independent claim 1. Further embodiments are defined in the corresponding dependent claims.

During practical operation of the device for measuring blood pressure as provided by embodiments of the present invention, for measuring the blood pressure, it only requires a user to contact the first electrode and the second electrode with his/her two hands respectively while contacting the photoelectric sensor with his/her one hand; as compared with the existing sphygmomanometer, it's simpler in operation without the need of wearing a wrist strap belt; moreover, the first electrode, the second electrode and the photoelectric sensor are all embedded at the surface of the housing so that it not only doesn't affect the acquisition of electrocardiosignal of the first electrode and the second electrode and the acquisition of pulse wave signal of the photoelectric sensor, but also achieves miniaturization and portability of the device for measuring blood pressure by integrating the first electrode, the second electrode and the photoelectric sensor within the housing, thereby satisfying the demand of user who requires for measurement of blood pressure whenever and wherever possible..

### BRIEF DESCRIPTION OF THE DRAWINGS

In order to clearly illustrate the technical solution of the embodiments of the present invention, the drawings of the embodiments will be briefly described in the following. Obviously, the drawings described as below merely refer to some embodiments of the present invention without limiting the present invention thereto.
Figs. 1and 2 are schematically structural diagrams illustrating a device for measuring blood pressure not falling under scope of the present invention as defined in the claims.
Figs. 3 and 4 are schematically structural diagrams illustrating a sphygmomanometerdevice for measuring blood pressure as provided by the present invention.
Fig. 5 is an oscillogram of electrocardiosignal and pulse wave signal as acquired by the device for measuring blood pressure as provided by embodiments of the present invention.

### DETAILED DESCRIPTION

Hereafter, particular implementations of a device for measuring blood pressure are further described in more details with reference to the appending drawings.

A shape or a dimension of respective components in the drawings is not intended to reflect an actual scale thereof but only to illustratively explain contents of the present invention.

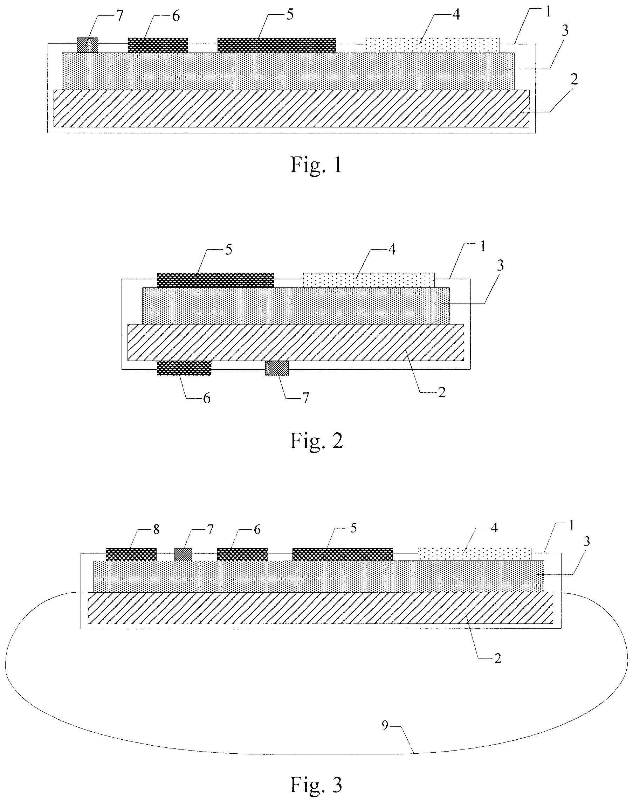

As illustrated in Figs. 1-4, a device for measuring blood pressure comprises a housing 1; a circuit board 2 and a power supply 3 that are disposed within the housing 1; a display 4 embedded at a surface of the housing 1; and a first electrode 5, a second electrode 6 and a photoelectric sensor 7 that are electrically connected to the circuit board 2, respectively; wherein the first electrode 5, the second electrode 6 and the photoelectric sensor 7 are embedded at a surface of the housing 1.

During practical operation of the above-mentioned device for measuring blood pressure, for measuring the blood pressure, it only requires a user to contact the first electrode and the second electrode with his/her two hands respectively while contacting the photoelectric sensor with his/her one hand; as compared with the existing sphygmomanometer, it's simpler in operation without the need of wearing a wrist strap belt; moreover, the first electrode, the second electrode and the photoelectric sensor are all embedded at the surface of the housing so that it not only doesn't affect the acquisition of electrocardiosignal of the first electrode and the second electrode and the acquisition of pulse wave signal of the photoelectric sensor but also achieves miniaturization and portability of the device for measuring blood pressure by integrating the first electrode, the second electrode and the photoelectric sensor within the housing, thereby satisfying the demand of user who requires for measurement of blood pressure whenever and wherever possible.

In particular implementation, for the above-mentioned device for measuring blood pressure, a processor and an amplifier that are electrically connected can be integrated on the circuit board. Particularly, the first electrode and the second electrode are electrically connected to two ends (a positive end and a negative end) of the amplifier respectively; the photoelectric sensor is electrically connected to the processor; the power supply is electrically connected to the processor, the amplifier, the photoelectric sensor and the display for supplying electrical power to the processor, the amplifier, the photoelectric sensor and the display (and/or Bluetooth).

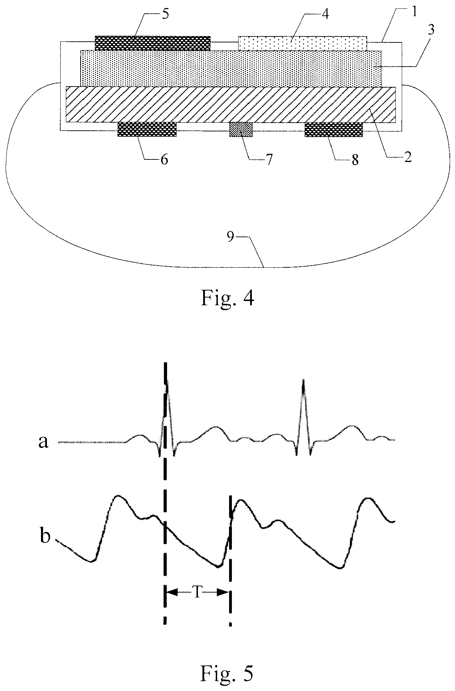

Hereinafter, particular working principle of the above-mentioned device for measuring blood pressure will be described in more details. The user contact the first electrode and the second electrode respectively with his/her two hands while contacting the photoelectric sensor with his/her one hand for a certain period of time (usually 10 seconds), so as to measure the blood pressure. During such operation, the amplifier is configured to acquire electrocardiosignal through the first electrode and the second electrode under a control of the processor and send the electrocardiosignal as acquired to the processor; the photoelectric sensor is configured to acquire pulse wave signal under a control of the processor and sends the pulse wave signal as acquired to the processor; the processor is configured to receive the electrocardiosignal sent by the amplifier and the pulse wave signal sent by the photoelectric sensor, recognize a peak point of the electrocardiosignal and a peak point of the pulse wave signal, determine a time difference between the peak point of the electrocardiosignal (as indicated by a curve a illustrated in Fig. 5) and the peak point of the pulse wave signal (as indicated by a curve b illustrated in Fig. 5) as a pulse transmission time (as indicated by T as illustrated in Fig. 5), substitute the pulse transmission time into an equation of blood pressure versus pulse transmission time as pre-stored to calculate a blood pressure value, and send the blood pressure value to the display; the display receives and displays the blood pressure value sent by the processor.

It should be explained that, in the above-mentioned device for measuring blood pressure, the equation of blood pressure versus pulse transmission time pre-stored in the processor can be obtained by way of calibration. In particular, measuring a standard blood pressure of a human body by using a standard mercury sphygmomanometer and measuring a current pulse transmission time of the human body by using the sphygmomanometer to be calibrated at the same time, so as to obtain a group of data of blood pressure versus pulse transmission time; measuring a plurality of groups of data of blood pressure versus pulse transmission time and linearly fitting the plurality of groups of data, so as to obtain the equation of blood pressure versus pulse transmission time for the sphygmomanometer to be calibrated; then inputting the equation into the processor.

During particular implementation, when a user measures blood pressure by using the above-mentioned device for measuring blood pressure, he/she contacts both the first electrode and the second electrode with his/her two hands respectively while contacting the photoelectric sensor with his/her one hand. Particularly, the user can choose to contact the first electrode and the photoelectric sensor with the same hand, and to contact the second electrode with the other hand; or, the user can choose to contact the second electrode and the photoelectric sensor with the same hand, and to contact the first electrode with the other hand; however, embodiments of the present invention are not limited thereto. Hereinafter the case where the user contacts the second electrode and the photoelectric sensor with the same hand and contacts the first electrode with the other hand will be described by way of example.

In some implementations, for the above-mentioned device for measuring blood pressure, in order for the convenience of the user to contact both the second electrode and the photoelectric sensor at the same time with the same hand, as illustrated in Figs. 1-4, the second electrode 6 and the photoelectric sensor 7 can be embedded at the same side of the housing 1, that is, a surface of the second electrode 6 and a surface of the photoelectric sensor 7 are exposed from the same side of the housing 1.

In some embodiments, for the above-mentioned device for measuring blood pressure, when the second electrode and the photoelectric sensor are embedded at the same side of the housing, as illustrated in Fig. 1, the first electrode 5 and the second electrode 6 can be embedded at the same side of the housing 1; in this way, the first electrode 5, the second electrode 6 and the photoelectric sensor 7 are all embedded at the same side of the housing 1, that is, a surface of the first electrode 5, a surface of the second electrode 6 and a surface of the photoelectric sensor 7 are all exposed from the same side of the housing 1; in this way, when measuring a blood pressure by using the above-mentioned device for measuring blood pressure, the user can press one of his/her hands on the first electrode 5 while pressing the other hand on both the second electrode 6 and the photoelectric sensor 7 for a certain period of time so that the display 4 of the device for measuring blood pressure can display a blood pressure value. At this time, in order for the convenience of the user to view the blood pressure value displayed on the display 4, as illustrated in Fig. 1, the display 4 can also be embedded at the side so that all of the first electrode 5, the second electrode 6, the photoelectric sensor 7 and the display 4 can be embedded at the same side of the housing 1, that is, a surface of the first electrode 5, a surface of the second electrode 6, a surface of the photoelectric sensor 7 and a surface of the display 4 are all exposed from the same side of the housing 1.

Of course, the surface of the display can be exposed from any side of the housing; in particular, it can be adapted appropriately according to actual conditions by comprehensively considering several factors such as the convenience for the user to view the blood pressure value and the reduction of the size of the device for measuring blood pressure as far as possible.

In some implementations, for the above-mentioned device for measuring blood pressure, when the second electrode and the photoelectric sensor are embedded at the same side of the housing, as illustrated in Fig. 2, the first electrode 5 and the second electrode 6 can be embedded at two opposite sides of the housing 1 respectively, that is, a surface of the first electrode 5 and a surface of the second electrode 6 are exposed from two opposite sides of the housing 1 respectively; in this way, the size of the device for measuring blood pressure can be further reduced. At this time, in order for the convenience of the user to view the blood pressure value displayed by the display 4 and for the reduction of the size of the device for measuring blood pressure, as illustrated in Fig. 2, the first electrode 5 and the display 4 can be embedded at the same side of the housing 1, that is, a surface of the first electrode 5 and a surface of the display 4 both are exposed from the same side of the housing 1.

Of course, in case where the second electrode and the photoelectric sensor are embedded at the same side of the housing, the first electrode and the second electrode can also be embedded at two adjacent sides of the housing 1 respectively, that is, a surface of the first electrode and a surface of the second electrode are exposed from two adjacent sides of the housing respectively; however, embodiments of the present invention are not limited thereto.

In some implementations, for the above-mentioned device for measuring blood pressure, in order to ensure that the user can fully contact the first electrode with his/her hand so as to guarantee an accuracy of the electrocardiosignal as acquired by the amplifier and also reduce the size of the device for measuring blood pressure as far as possible for achieving miniaturization and portability thereof, a length of the surface of the first electrode exposed from the housing can be controlled to be within a range from 15 mm to 25 mm, a width of the surface of the first electrode exposed from the housing can be controlled to be within a range from 8 mm to 12 mm, and a thickness of the first electrode can be controlled to be within a range from 1 mm to 1.5 mm.

In some implementations, for the above-mentioned device for measuring blood pressure, in order to ensure that the user can fully contact the second electrode with his/her hand so as to guarantee an accuracy of the electrocardiosignal as acquired by the amplifier and also reduce the size of the device for measuring blood pressure as far as possible for achieving miniaturization and portability thereof, a length of the surface of the second electrode exposed from the housing can be controlled to be within a range from 15 mm to 25 mm, a width of the surface of the second electrode exposed from the housing can be controlled to be within a range from 8 mm to 12 mm, and a thickness of the second electrode can be controlled to be within a range from 1 mm to 1.5 mm.

In some implementations, for the above-mentioned device for measuring blood pressure, in order to ensure that the user can fully contact the photoelectric sensor with his/her hand so as to guarantee an accuracy of the pulse wave signal as acquired by the photoelectric sensor and also reduce the size of the device for measuring blood pressure as far as possible for achieving miniaturization and portability thereof, a length of the surface of the photoelectric sensor exposed from the housing can be controlled to be within a range from 5 mm to 6 mm, a width of the surface of the photoelectric sensor exposed from the housing can be controlled to be within a range from 2 mm to 2.5 mm, and a thickness of the photoelectric sensor can be controlled to be within a range from 1 mm to 1.5 mm.

In the invention, as illustrated in Fig. 3 and Fig. 4, the above-mentioned device for measuring blood pressure as embodiments of the present invention further comprise a third electrode 8 electrically connected to the circuit board 2; in particular, the third electrode 8 is electrically connected to one end of the amplifier connected to the second electrode 6 so that the third electrode 8 is used as a feedback electrode serving for suppressing common-mode interference, thereby improving an accuracy of the electrocardio signal as acquired by the amplifier; moreover, the third electrode 8 and the second electrode 6 are embedded at the same side of the housing 1, that is, a surface of the third electrode 8 and a surface of the second electrode 6 are exposed from the same side of the housing 1 so as to facilitate the user to contact the third electrode 8 and the second electrode 6 with one hand at the same time.

In some implementations, for the above-mentioned device for measuring blood pressure n, in order to ensure that the user can fully contact the third electrode with his/her hand so as to guarantee an accuracy of electrocardiosignal as acquired by the amplifier and also reduce the size of the device for measuring blood pressure as far as possible for achieving miniaturization and portability thereof, a length of the surface of the third electrode exposed from the housing can be controlled to be within a range from 15 mm to 25 mm, a width of the surface of the third electrode exposed from the housing can be controlled to be within a range from 8 mm to 12 mm, and a thickness of the third electrode can be controlled to be within a range from 1 mm to 1.5 mm.

In some embodiments, as illustrated in Fig. 3 and Fig. 4, the above-mentioned device for measuring blood pressure as embodiments of the present invention can further comprise a wrist strap belt 9 connected to the housing 1; in this way, the user can directly wear the device for measuring blood pressure on his/her wrist for convenience of measuring the blood pressure whenever and wherever possible. For example, when measuring the blood pressure with the device for measuring blood pressure as illustrated in Fig. 3, the user can take off the device for measuring blood pressure from the wrist, then presses one hand on the first electrode 5 while pressing the other hand on the second electrode 6, the photoelectric sensor 7 and the third electrode 8 for a certain period of time so that the display 4 can display a blood pressure value; when measuring the blood pressure with the device for measuring blood pressure as illustrated in Fig. 4, it's not necessary for the user to take off the device for measuring blood pressure from the wrist but only needs to directly press the hand without wearing the device for measuring blood pressure on the first electrode 5 and to allow the second electrode 5, the third electrode 8 and the photoelectric sensor 7 all contacting the wrist wearing the device for measuring blood pressure for a certain period of time so that the display 4 can display a blood pressure value; in this way, the operation of the device for measuring blood pressure is further simplified. It should be noted that, when wearing the device for measuring blood pressure as illustrated in Fig. 4, it has to allow the exposed surface of the second electrode 6, the exposed surface of the third electrode 8 and the exposed surface of the photoelectric sensor 7 all facing to the wrist of the user.

During particular implementation, for the above-mentioned device for measuring blood pressure, the processor can be an ARM processor capable of controlling an acquisition of signal (e.g., controlling a sampling rate and a signal magnification of the amplifier; controlling a sampling rate and an illumination intensity of the photoelectric sensor, etc.) and reading and processing data in real time.

During particular implementation, for the above-mentioned device for measuring blood pressure, the power supply can be a rechargeable battery, such as, a lithium battery.

It should be explained that, for the above-mentioned device for measuring blood pressure, the photoelectric sensor can preferably be a reflective photoelectric sensor. Because both of a luminotron and a photoreceiver of the reflective photoelectric are located at the same side of the user's hand, the reflective photoelectric sensor can be integrated within the housing with only the surface of the reflective photoelectric sensor being exposed, so as to ensure achieving miniaturization and portability of the device for measuring blood pressure.

It should be explained that the above-mentioned device for measuring blood pressure is not limited to a structure which displays the blood pressure value through a display, but also can utilize a Bluetooth in place of the display. In particular, the Bluetooth can send the blood pressure value calculated by the processor to a mobile phone or a computer of the user through which the blood pressure value can be read; in this way, the size of the device for measuring blood pressure can be further reduced so that the device for measuring blood pressure is smaller and more portable.

Hereinafter particular dimensions of the above-mentioned device for measuring blood pressure as provided by embodiments of the present invention will be described in more details with reference to the device for measuring blood pressure as illustrated in Fig. 4 by way of example. According to particular dimensions of the first electrode, the second electrode, the photoelectric sensor and the third electrode, by considering that a thickness of a circuit board and a lithium battery usually are 3 mm and 4 mm respectively, it can be deduced that the above-mentioned device for measuring blood pressure as provided by embodiments of the present invention has a length of 35 mm to 45 mm, a width of 28 mm to 35 mm and a thickness of 9 mm to 15 mm; more particularly, the length is about 40 mm, the width is about 30 mm, and the thickness is about 10mm. The above-mentioned device for measuring blood pressure as provided by embodiments of the present invention is small in size and convenient for portability.

The embodiments of the present invention provide a device for measuring blood pressure, during practical operation, in order to measure the blood pressure, it only requires a user to contact the first electrode and the second electrode with his/her two hands respectively while contacting the photoelectric sensor with his/her one hand; as compared with the existing sphygmomanometer, it's simpler in operation without the need of wearing a wrist strap belt; moreover, the first electrode, the second electrode and the photoelectric sensor are all embedded at the surface of the housing so that it not only doesn't affect the acquisition of electrocardiosignal of the first electrode and the second electrode and the acquisition of pulse wave signal of the photoelectric sensor but also achieves miniaturization and portability of the sphygmomanometer by integrating the first electrode, the second electrode and the photoelectric sensor within the housing, thereby satisfying the demand of user who requires for measurement of blood pressure whenever and wherever possible.

## Claims

1. A device for measuring blood pressure, comprising:
a housing (1);
a circuit board (2) and a power supply (3) that are disposed within the housing (1); a display (4) embedded at the surface of the housing (1); and
a first electrode (5), a second electrode (6) and a photoelectric sensor (7) that are electrically connected to the circuit board (2), respectively; wherein,
the first electrode (5), the second electrode (6) and the photoelectric sensor (7) are embedded at a surface of the housing (1);
a processor and an amplifier which are integrated on the circuit board (2),
the first electrode (5) and the second electrode (6) are electrically connected to two ends of the amplifier, respectively;
the photoelectric sensor (7) is electrically connected to the processor;
**characterized in that**
the device further comprises a third electrode (8) electrically connected to the circuit board (2), wherein the second electrode (6), the third electrode (8) and the photoelectric sensor (7) are embedded at a same side of the housing (1), and the third electrode (8) is electrically connected to one end of the amplifier connected to the second electrode (6) so that the third electrode (8) is used as a feedback electrode serving for suppressing common-mode interference.

2. The device of claim 1, wherein the second electrode (6) and the photoelectric sensor (7) are embedded at a same side of the housing (1).

3. The device of claim 1 or 2, wherein the first electrode (5) and the second electrode (6) are embedded at a same side of the housing (1).

4. The device of claim 2, wherein the first electrode and the second electrode are embedded at two opposite sides of the housing, respectively.

5. The device of any one of claims 1-4, wherein a surface of the first electrode (5) exposed from the housing (1) has a length of 15 mm to 25 mm, the surface of the first electrode (5) exposed from the housing (1) has a width of 8 mm to 12 mm, and the first electrode (5) has a thickness of 1 mm to 1.5 mm.

6. The device of any one of claims 1-4, wherein a surface of the second electrode (6) exposed from the housing (1) has a length of 15 mm to 25 mm, the surface of the second electrode (6) exposed from the housing (1) has a width of 8 mm to 12 mm, and the second electrode (6) has a thickness of 1 mm to 1.5 mm.

7. The device of any one of claims 1-4, wherein a surface of the photoelectric sensor (7) exposed from the housing (1) has a length of 5 mm to 6 mm, the surface of the photoelectric sensor (7) exposed from the housing (1) has a width of 2 mm to 2.5 mm, and the photoelectric sensor (7) has a thickness of 1 mm to 1.5 mm.

8. The device of any one of claims 1-4, wherein the third electrode (8) and the second electrode (6) are embedded at a same side of the housing (1).

9. The device of claim 8, wherein a surface of the third electrode exposed from the housing has a length of 15 mm to 25 mm, the surface of the third electrode exposed from the housing has a width of 8 mm to 12 mm, and the third electrode has a thickness of 1 mm to 1.5 mm.

10. The device of any one of claims 1-4, further comprising a wrist strap belt (9) connected to the housing (1).

11. The device of any of the preceding claims, wherein
the processor is configured to store an equation of a blood pressure versus a pulse transmission time, the equation reflecting a relationship between the blood pressure and the pulse transmission time.

12. The device of claim 11, further configured to realize functions as below:
the amplifier is configured to acquire an electrocardiosignal through the first electrode (5) and the second electrode (6) under a control of the processor and send the electrocardiosignal as acquired to the processor;
the photoelectric sensor (7) is configured to acquire pulse wave signal under a control of the processor and sends the pulse wave signal as acquired to the processor;
the processor is configured to receive the electrocardiosignal sent by the amplifier and the pulse wave signal sent by the photoelectric sensor (7), recognize a peak point of the electrocardiosignal and a peak point of the pulse wave signal, determine a time difference between the peak point of the electrocardiosignal and the peak point of the pulse wave signal as a pulse transmission time, substitute the pulse transmission time into the equation of blood pressure versus pulse transmission time to calculate a blood pressure value, and perform at least one of the following operations:
send the blood pressure value to the display (14) which receives and displays the blood pressure value sent by the processor; and
send the blood pressure value to an external receiving device through Bluetooth, wherein the external receiving device is at least one selected from a mobile phone and a computer.

13. The device of any one of the preceding claims, wherein the device has a length of 35 mm to 45 mm, a width of 28 mm to 35 mm, and a thickness of 9 mm to 15 mm.

## Patentansprüche

1. Vorrichtung zur Messung des Blutdrucks, mit:
einem Gehäuse (1);
einer Leiterplatte (2) und einer Stromversorgung (3), die innerhalb des Gehäuses (1) angeordnet sind;
einer Anzeige (4), die in die Oberfläche des Gehäuses (1) eingebettet ist; und
einer ersten Elektrode (5), einer zweiten Elektrode (6) und einem photoelektrischen Sensor (7), die jeweils elektrisch mit der Leiterplatte (2) verbunden sind; wobei,
die erste Elektrode (5), die zweite Elektrode (6) und der fotoelektrische Sensor (7) an einer Oberfläche des Gehäuses (1) eingebettet sind;
ein Prozessor und ein Verstärker auf der Leiterplatte (2) integriert,
die erste Elektrode (5) und die zweite Elektrode (6) jeweils mit zwei Enden des Verstärkers elektrisch verbunden sind;
der photoelektrische Sensor (7) elektrisch mit dem Prozessor verbunden ist;
**dadurch gekennzeichnet, dass**
die Vorrichtung ferner eine dritte Elektrode (8) aufweist, die elektrisch mit der Leiterplatte (2) verbunden ist, wobei die zweite Elektrode (6), die dritte Elektrode (8) und der fotoelektrische Sensor (7) auf derselben Seite des Gehäuses (1) eingebettet sind und die dritte Elektrode (8) elektrisch mit einem Ende des Verstärkers verbunden ist, der mit der zweiten Elektrode (6) verbunden ist, sodass die dritte Elektrode (8) als Rückkopplungselektrode verwendet wird, die zur Unterdrückung von Gleichtaktstörungen dient.

2. Vorrichtung nach Anspruch 1, wobei die zweite Elektrode (6) und der photoelektrische Sensor (7) auf derselben Seite des Gehäuses (1) eingebettet sind.

3. Vorrichtung nach Anspruch 1 oder 2, wobei die erste Elektrode (5) und die zweite Elektrode (6) auf der gleichen Seite des Gehäuses (1) eingebettet sind.

4. Vorrichtung nach Anspruch 2, bei der die erste Elektrode und die zweite Elektrode jeweils an zwei gegenüberliegenden Seiten des Gehäuses eingebettet sind.

5. Vorrichtung nach einem der Ansprüche 1-4, wobei eine Oberfläche der ersten Elektrode (5), die aus dem Gehäuse (1) herausragt, eine Länge von 15 mm bis 25 mm hat, die Oberfläche der ersten Elektrode (5), die aus dem Gehäuse (1) herausragt, eine Breite von 8 mm bis 12 mm hat und die erste Elektrode (5) eine Dicke von 1 mm bis 1,5 mm hat.

6. Vorrichtung nach einem der Ansprüche 1-4, wobei eine Oberfläche der zweiten Elektrode (6), die aus dem Gehäuse (1) herausragt, eine Länge von 15 mm bis 25 mm hat, die Oberfläche der zweiten Elektrode (6), die aus dem Gehäuse (1) herausragt, eine Breite von 8 mm bis 12 mm hat und die zweite Elektrode (6) eine Dicke von 1 mm bis 1,5 mm hat.

7. Vorrichtung nach einem der Ansprüche 1-4, wobei eine Oberfläche des photoelektrischen Sensors (7), die aus dem Gehäuse (1) herausragt, eine Länge von 5 mm bis 6 mm hat, die Oberfläche des photoelektrischen Sensors (7), die aus dem Gehäuse (1) herausragt, eine Breite von 2 mm bis 2,5 mm hat und der photoelektrische Sensor (7) eine Dicke von 1 mm bis 1,5 mm hat.

8. Vorrichtung nach einem der Ansprüche 1-4, wobei die dritte Elektrode (8) und die zweite Elektrode (6) auf derselben Seite des Gehäuses (1) eingebettet sind.

9. Vorrichtung nach Anspruch 8, wobei eine Oberfläche der dritten Elektrode, die aus dem Gehäuse herausragt, eine Länge von 15 mm bis 25 mm hat, die Oberfläche der dritten Elektrode, die aus dem Gehäuse herausragt, eine Breite von 8 mm bis 12 mm hat und die dritte Elektrode eine Dicke von 1 mm bis 1,5 mm hat.

10. Vorrichtung nach einem der Ansprüche 1-4, die ferner einen Handgelenk-Gurtband (9) aufweist, das mit dem Gehäuse (1) verbunden ist.

11. Vorrichtung nach einem der vorhergehenden Ansprüche, wobei
der Prozessor konfiguriert ist, eine Gleichung zwischen Blutdruck und Pulsübertragungszeit zu speichern, wobei die Gleichung eine Beziehung zwischen dem Blutdruck und der Pulsübertragungszeit wiedergibt.

12. Vorrichtung nach Anspruch 11, weiter konfiguriert, um die folgenden Funktionen zu realisieren:
der Verstärker ist konfiguriert, ein Elektrokardiosignal über die erste Elektrode (5) und die zweite Elektrode (6) unter Steuerung des Prozessors zu erfassen und das erfasste Elektrokardiosignal an den Prozessor zu senden;
der photoelektrische Sensor (7) ist konfiguriert, unter Steuerung des Prozessors ein Pulswellensignal zu erfassen, und das erfasste Pulswellensignal an den Prozessor zu senden;
der Prozessor ist konfiguriert, das vom Verstärker gesendete Elektrokardiosignal und das vom photoelektrischen Sensor (7) gesendete Pulswellensignal zu empfangen, einen Spitzenpunkt des Elektrokardiosignals und einen Spitzenpunkt des Pulswellensignals zu erkennen, eine Zeitdifferenz zwischen dem Spitzenpunkt des Elektrokardiosignals und dem Spitzenpunkt des Pulswellensignals als Pulsübertragungszeit zu bestimmen, die Pulsübertragungszeit in die Gleichung des Blutdrucks gegenüber der Pulsübertragungszeit einzusetzen, um einen Blutdruckwert zu berechnen, und mindestens eine der folgenden Operationen durchzuführen:
Senden des Blutdruckwerts an das Display (14), das den vom Prozessor gesendeten Blutdruckwert empfängt und anzeigt; und
Senden des Blutdruckwerts an ein externes Empfangsgerät über Bluetooth, wobei das externe Empfangsgerät ein Mobiltelefon und/oder ein Computer ist.

13. Vorrichtung nach einem der vorhergehenden Ansprüche, wobei die Vorrichtung eine Länge von 35 mm bis 45 mm, eine Breite von 28 mm bis 35 mm und eine Dicke von 9 mm bis 15 mm aufweist.

## Revendications

1. Dispositif pour mesurer la pression artérielle, comprenant :
un boîtier (1) ;
une carte de circuit (2) et une alimentation électrique (3) qui sont disposées à l'intérieur du boîtier (1) ;
un affichage (4) qui est encastré au niveau de la surface du boîtier (1) ; et
une première électrode (5), une deuxième électrode (6) et un capteur photoélectrique (7) qui sont respectivement connectés électriquement à la carte de circuit (2) ;
dans lequel :
la première électrode (5), la deuxième électrode (6) et le capteur photoélectrique (7) sont encastrés au niveau d'une surface du boîtier (1) ;
un processeur et un amplificateur qui sont intégrés sur la carte de circuit (2),
la première électrode (5) et la deuxième électrode (6) sont respectivement connectées électriquement à deux extrémités de l'amplificateur ;
le capteur photoélectrique (7) est connecté électriquement au processeur,
**caractérisé en ce que** :
le dispositif comprend en outre une troisième électrode (8) qui est connectée électriquement à la carte de circuit (2), dans lequel la deuxième électrode (6), la troisième électrode (8) et le capteur photoélectrique (7) sont encastrés au niveau d'un même côté du boîtier (1), et la troisième électrode (8) est connectée électriquement à une extrémité de l'amplificateur qui est connectée à la deuxième électrode (6) de telle sorte que la troisième électrode (8) soit utilisée en tant qu'électrode de rétroaction qui sert à supprimer l'interférence de mode commun.

2. Dispositif selon la revendication 1, dans lequel la deuxième électrode (6) et le capteur photoélectrique (7) sont encastrés au niveau d'un même côté du boîtier (1).

3. Dispositif selon la revendication 1 ou 2, dans lequel la première électrode (5) et la deuxième électrode (6) sont encastrées au niveau d'un même côté du boîtier (1).

4. Dispositif selon la revendication 2, dans lequel la première électrode et la deuxième électrode sont respectivement encastrées au niveau de deux côtés opposés du boîtier.

5. Dispositif selon l'une quelconque des revendications 1 à 4, dans lequel une surface de la première électrode (5) qui est exposée depuis le boîtier (1) présente une longueur de 15 mm à 25 mm, la surface de la première électrode (5) qui est exposée depuis le boîtier (1) présente une largeur de 8 mm à 12 mm, et la première électrode (5) présente une épaisseur de 1 mm à 1,5 mm.

6. Dispositif selon l'une quelconque des revendications 1 à 4, dans lequel une surface de la deuxième électrode (6) qui est exposée depuis le boîtier (1) présente une longueur de 15 mm à 25 mm, la surface de la deuxième électrode (6) qui est exposée depuis le boîtier (1) présente une largeur de 8 mm à 12 mm, et la deuxième électrode (6) présente une épaisseur de 1 mm à 1,5 mm.

7. Dispositif selon l'une quelconque des revendications 1 à 4, dans lequel une surface du capteur photoélectrique (7) qui est exposée depuis le boîtier (1) présente une longueur de 5 mm à 6 mm, la surface du capteur photoélectrique (7) qui est exposée depuis le boîtier (1) présente une largeur de 2 mm à 2,5 mm, et le capteur photoélectrique (7) présente une épaisseur de 1 mm à 1,5 mm.

8. Dispositif selon l'une quelconque des revendications 1 à 4, dans lequel la troisième électrode (8) et la deuxième électrode (6) sont encastrées au niveau d'un même côté du boîtier (1).

9. Dispositif selon la revendication 8, dans lequel une surface de la troisième électrode qui est exposée depuis le boîtier présente une longueur de 15 mm à 25 mm, la surface de la troisième électrode qui est exposée depuis le boîtier présente une largeur de 8 mm à 12 mm, et la troisième électrode présente une épaisseur de 1 mm à 1,5 mm.

10. Dispositif selon l'une quelconque des revendications 1 à 4, comprenant en outre une sangle de dragonne (9) connectée au boîtier (1).

11. Dispositif selon l'une quelconque des revendications précédentes, dans lequel :
le processeur est configuré pour stocker une équation d'une pression artérielle en fonction d'un temps de transmission des impulsions, l'équation reflétant une relation entre la pression artérielle et le temps de transmission des impulsions.

12. Dispositif selon la revendication 11, configuré en outre pour réaliser les fonctions telles que ci-dessous :
l'amplificateur est configuré pour acquérir un signal électro-cardiaque par l'intermédiaire de la première électrode (5) et de la deuxième électrode (6) sous la commande du processeur et pour envoyer le signal électro-cardiaque tel qu'acquis au processeur ;
le capteur photoélectrique (7) est configuré pour acquérir un signal d'onde des impulsions en tant que temps de transmission des impulsions sous la commande du processeur et pour envoyer le signal d'onde des impulsions tel qu'acquis au processeur ; et
le processeur est configuré pour recevoir le signal électro-cardiaque envoyé par l'amplificateur et le signal d'onde des impulsions envoyé par le capteur photoélectrique (7), pour reconnaître un point de crête du signal électro-cardiaque et un point de crête du signal d'onde des impulsions, pour déterminer une différence temporelle entre le point de crête du signal électro-cardiaque et le point de crête du signal d'onde des impulsions en tant que temps de transmission des impulsions, pour substituer le temps de transmission des impulsions à l'intérieur de l'équation de la pression artérielle en fonction du temps de transmission des impulsions afin de calculer une valeur de pression artérielle, et pour réaliser au moins l'une des opérations suivantes :
envoyer la valeur de pression artérielle sur l'affichage (14) qui reçoit et affiche la valeur de pression artérielle envoyée par le processeur ; et
envoyer la valeur de pression artérielle sur un dispositif de réception externe par l'intermédiaire de Bluetooth, dans lequel le dispositif de réception externe est au moins un dispositif sélectionné parmi un téléphone mobile et un ordinateur.

13. Dispositif selon l'une quelconque des revendications précédentes, dans lequel le dispositif présente une longueur de 35 mm à 45 mm, une largeur de 28 mm à 35 mm et une épaisseur de 9 mm à 15 mm.
